# EUROPÄISCHE PATENTSCHRIFT

(11) **EP 0 393 427 B1**
(45) Veröffentlichungstag und Bekanntmachung des Hinweises auf die Patenterteilung: **02.11.1994**
(21) Anmeldenummer: 90106507.8
(22) Anmeldetag: 05.04.1990
(51) Int. Cl.: A61B 5/22, G01L 3/10, G01L 5/00

(54) **Vorrichtung zur Messung von Antriebsmomenten von Rädern**
Device for measuring driving torques on wheels
Dispositif pour la mesure des couples d'entraînement des roues

(30) Priorität: 19.04.1989 DE 3912883
(43) Veröffentlichungstag der Anmeldung: 24.10.1990
(73) Patentinhaber: Look S.A., 58004 Nevers cedex (FR)
(72) Erfinder: Mercat, Jean-Pierre, F-37110 Chateau-Renault (FR)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- DE-A- 3 150 149
- DE-A- 3 726 148
- DE-A- 3 813 681
- DE-B- 2 450 780

## Beschreibung

Die Erfindung betrifft eine Vorrichtung zur Messung des auf ein antreibbares Rad, insbesondere auf das Antriebsrad eines Fahrrades ausgeübten Antriebsmomentes und/oder damit verbundener Größen von der im Oberbegriff des Patentanspruchs 1 angegebenen Art.

Zur Verbesserung der körperlichen Leistungsfähigkeit wird das Fahrrad im Bereich des Sports zunehmend als spezielles Trainingsgerät verwendet, und außerdem werden Fahrräder, insbesondere in stationärer Ausführungsform zunehmend zur Behandlung und Rehabilitation von Patienten mit Herz-Kreislauferkrankungen eingesetzt. Bei der Durchführung solcher Trainings- und Behandlungsprogramme ist es wichtig, Informationen bezüglich der vom jeweiligen Sportler bzw. Patienten entwickelten Leistung und verbrauchten Energie zu erhalten, da es diese Informationen ermöglichen, Rückschlüsse auf Trainings- oder Behandlungsergebnisse zu ziehen.

Aus der DE-A1 31 50 149 ist es bereits bekannt, eine weitgehend verlustlose Leistungsmessung dadurch zu erreichen, daß zwischen einem angetriebenen Getrieberad und einem anzutreibenden Bauteil eine elastische Verbindung vorgesehen ist und der Drehwinkel gemessen wird, der sich beim Aufbringen von Antriebskräften auf das Getrieberad durch die Relativverdrehung zwischen Getrieberad und anzutreibendem Bauteil einstellt. Mittels zweier Meßwertaufnehmer werden dabei Impulsfolgen erzeugt, wobei der zeitliche Abstand zwischen den Impulsen dieser Meßwertaufnehmer als Maß für die übertragene Kraft ausgewertet wird.
Nachteilig bei dieser bekannten Vorrichtung ist vor allem, daß aufgrund der Schwierigkeit der Erfassung der sehr kleinen Verdrehungswinkel, die im Maximum 1 bis 2° betragen, die erzielbaren Meßgenauigkeiten sehr zu wünschen übrig lassen.

Aufgabe der Erfindung ist es daher, eine Vorrichtung der eingangs angegebenen Art in der Weise auszubilden, daß das jeweilige Antriebsmoment und/oder damit verbundene Größen auf einfache, störungssichere und ohne Auftreten zusätzlicher Reibungseffekte vor sich gehenden Weise äußerst genau und in einem großen Meßbereich erfaßt werden können.

Diese Aufgabe wird im wesentlichen durch die im kennzeichnenden Teil des Patentanspruches 1 angegebenen Merkmale gelöst.

Durch die Schaffung einer mechanischen Übersetzung wird der sich vom jeweiligen Antriebsmoment abhängige Verdrehungswinkel zwischen Antriebsachse und Radnabe wesentlich vergrößert, so daß die über entsprechende Sensoren erfolgende Meßwertbildung mit deutlich erhöhter Auflösung und erhöhter Genauigkeit erfolgen kann und damit die jeweiligen Auswerteergebnisse erhöhte Aussagekraft erhalten.

Als Übersetzungsorgan wird vorzugsweise ein Schwenkhebel verwendet, der in ein ringscheibenförmig ausgebildetes und durch eine Feder in einer Richtung gegen diesen Schwenkhebel vorgespanntes Trägerelement eingreift und dieses Trägerelement entsprechend übersetzt in Abhängigkeit von der Relativverdrehung zwischen Antriebsachse und Radnabe verschwenkt. Damit werden auch in diesem Trägerelement gehalterte Meßwertgeber über einen entsprechenden Winkel verschwenkt, was wiederum mittels eines Sensors erfaßbar ist, der zweckmäßigerweise in einer Ausnehmung der festen Achse angeordnet ist.

Nach einer bevorzugten Ausgestaltung der Erfindung ist der Schwenkhebel mit einem Ende an einer nabenfesten Achse gelagert, durchsetzt eine Öffnung in der Antriebsachse und liegt mit dem anderen Ende an dem verschwenkbaren Trägerelement an. Dabei steht eine Begrenzungswand der Öffnung aufgrund der Federvorspannung des Trägerelements stets in Verbindung mit dem Schwenkhebel und bildet somit das eigentliche Schwenkhebelbetätigungsorgan.

Da das Lager für den Schwenkhebel vorzugsweise in einem mit der Radnabe fest verbundenen Führungsstück ausgebildet und unmittelbar benachbart der Antriebsachse angeordnet ist, führen bereits sehr geringe Relativverdrehungen zwischen Antriebsachse und Radnabe zu einem vergleichsweise großen Verschwenkweg des freien Endes des Schwenkhebels und damit auch zu einer entsprechend großen Übersetzung.

Besonders vorteilhaft ist es, diejenige Seitenfläche der Ausnehmung im Trägerelement, an der der Schwenkhebel anliegt, und/oder das freie Ende dieses Schwenkhebels als Steuerprofilfläche auszubilden, da auf diese Weise die Übersetzungscharakteristik vorgegeben und insbesondere auch eine lineare Übersetzungscharakteristik festlegbar ist.

Als Meßwertgeber werden vorzugsweise mehrere auf dem gleichen Radius liegende, über den Umfang verteilte Permanentmagnete verwendet, und als zugeordnete Sensoren werden Reedkontakte vorgesehen. Auf diese Weise erhält man eine einfache, kostengünstige, störungsunanfällige und auch raumsparend zu realisierende Anordnung. Ebenfalls in besonders vorteilhafter Weise läßt sich der Meßwertgeber in Form eines insbesondere flächig ausgebildeten Widerstandes mit zugehörigem Abtastelement realisieren.

Weitere vorteilhafte Ausgestaltungen der Erfindung sind in abhängigen Ansprüchen angegeben.

Die Erfindung wird nachfolgend anhand eines Ausführungsbeispiels unter Bezugnahme auf die Zeichnung erläutert; in der Zeichnung zeigt:
- Fig. 1: eine schematische Axialschnittdarstellung einer gemäß der Erfindung ausgebildeten Nabeneinheit für das Hinterrad eines Fahrrads,
- Fig. 2A, 2B und 2C: Schnittdarstellungen entsprechend der Schnittlinie S-S in Fig. 1 für verschiedene Betriebszustände, und
- Fig. 3: eine schematische Darstellung zur Erläuterung der Signalgewinnungs- und Verarbeitungsanordnung analog der Vorrichtung nach Fig. 1.

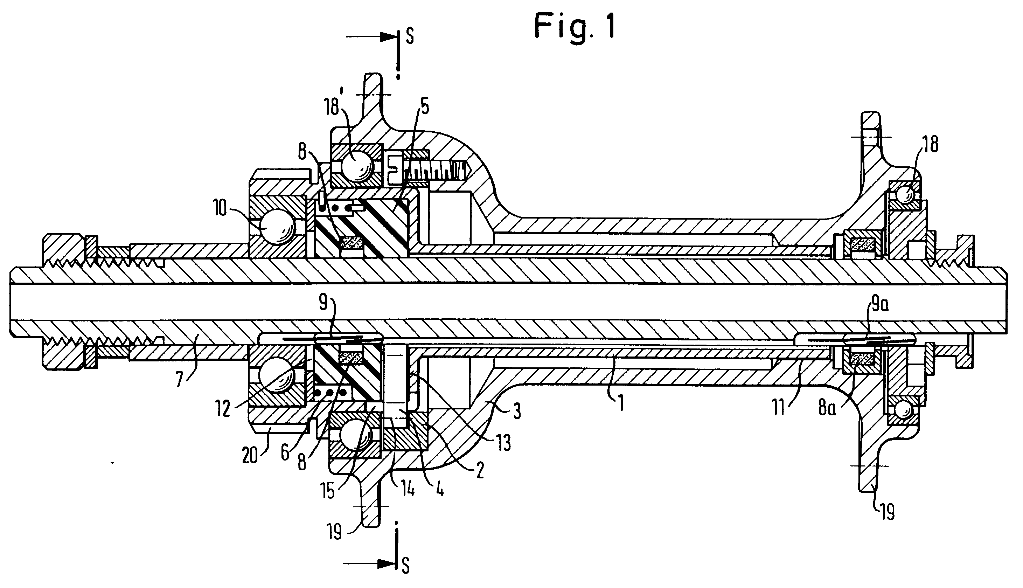

Fig. 1 zeigt eine feste Achse 7 zur Verspannung in der Gabel eines Hinterrads eines Fahrrads.
Auf dieser festen Achse 7 ist einerseits eine Radnabe 3 mit üblichen Speichenbefestigungsscheiben 19 sowie eine Antriebsachse 1 drehbar gelagert.
Die zur festen Achse 7 koaxiale Antriebsachse 1 ist mit einem Ende über ein Kugellager 10 bezüglich der festen Achse 7 abgestützt und mit ihrem anderen Ende über eine vorzugsweise durch Verkeilung gebildete Festverbindung 11 mit der Radnabe 3 drehfest verbunden.

Das außenliegende freie Ende der Antriebsachse 1 ist mit einer Aufnahme 20 für über einen Freilauf zu befestigende Antriebszahnkränze versehen.

Die Radnabe 3 ist bezuglich der festen Achse 7 und bezüglich des außenliegenden Endes der Antriebsachse 1 über Kugellager 18 bzw. 18′ abgestützt.

Anstelle des am außenliegenden Ende der Antriebsachse gelegenen Kugellagers 18′ wird vorzugsweise ein aus Kunststoff bestehendes, besonders kostengünstig zu fertigendes Doppelringelement verwendet, bei dem die beiden als Stützringe wirkenden Ringe über verformbare Radialstege miteinander verbunden sind. Dieses einteilige, ein teureres Kugellager funktionsmäßig voll ersetzende Element erbringt ohne Schwierigkeiten die erforderliche Relativverdrehung.

Radial innerhalb des zwischen Radnabe 3 und Antriebsachse 1 vorgesehenen Kugellagers 18 ist zwischen der festen Achse 7 und der Radnabe 3 ein Führungsraum 12 ausgebildet, in dem ein ringscheibenförmiges Trägerelement 5 angeordnet ist. Dieses Trägerelement 5 ist um die feste Achse 7 verschwenkbar und in einer Richtung durch eine Torsionsfeder 6 vorgespannt. Diese Torsionsfeder ist mit einem Ende am Trägerelement 5 und mit ihrem anderen Ende an der Radnabe 3 befestigt. In entsprechenden, festachsenseitig gelegenen Ausnehmungen des Trägerelements 5 sind mehrere über den Umfang verteilt angeordnete Permanentmagnete 8 befestigt, insbesondere durch Verkleben.

Im Bereich des Trägerelements 5 ist zwischen der Radnabe 3 und der Antriebsachse 1 ein mit der Radnabe 3 fest verbundenes Führungsstück 2 angebracht, in dem um eine Achse 14 schwenkbar ein als Schwenkhebel ausgebildetes Übersetzungsorgan 4 gelagert ist. Dieser Schwenkhebel 4 greift in eine Ausnehmung 13 des Trägerelements 5 ein.

Dem Permanentmagneten 8 im Trägerorgan 5 gegenüberliegend ist in einer entsprechenden Ausnehmung der festen Achse 7 ein Reedkontakt 9 angeordnet, der bei jedem Vorbeilaufen eines Permanentmagneten 8 schaltet und jeweils einen eine Einschaltflanke und eine Ausschaltflanke aufweisenden Impuls erzeugt.

Angrenzend an die Festverbindung 11 zwischen Antriebsachse 1 und Radnabe 3 ist in der Radnabe 3 eine Halterung für mehrere über den Umfang verteilte Permanentmagneten 8a vorgesehen, wobei diese Permanentmagnete 8a mit den im Trägerelement 5 vorgesehenen Permanentmagneten 8 ausgerichtet sind bzw. in den gleichen Radialebenen gelegen sind. Auch diesem Permanentmagneten 8a ist ein in einer Ausnehmung der festen Achse 7 fixierter Reedkontakt 9a zugeordnet.

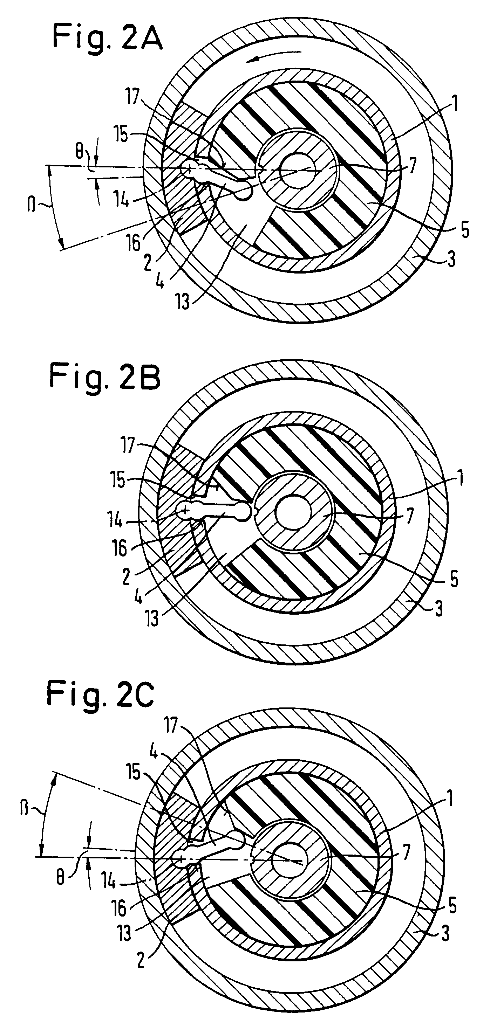

Fig. 2A zeigt einen Schnitt entsprechend der Schnittlinie S-S in Fig. 1, und zwar für den Fall eines Antriebsmomentes Null.

Das sich zwischen der festen Achse 7 und der Antriebsachse 1 befindende Trägerelement 5 ist in Pfeilrichtung durch eine Torsionsfeder vorgespannt und liegt somit stets an dem Schwenkhebel 4 an. Dieser Schwenkhebel 4 ist in einer Paßausnehmung des mit der Radnabe 3 fest verbundenen Führungsstückes 2 schwenkbar gelagert und erstreckt sich durch eine Öffnung 15 der Antriebsachse 1 in die Ausnehmung 13 des Trägerelements 5, wobei er mit seinem freien Ende an einer Steuerprofilfläche 17 dieses Trägerelements 5 anliegt. Durch die Federvorspannung des Trägerelements 5 liegt der Schwenkhebel 4 ferner stets an einer Begrenzungswand 16 der Öffnung 15 in der Antriebsachse 1 an, und diese Begrenzungswand 16 bildet das eigentliche Betätigungsorgan für den Schwenkhebel 4.

Der Abstand zwischen dem wirksamen Angriffspunkt der Begrenzungswand 16 am Schwenkhebel 4 zur nabenfesten Achse 14 ist sehr gering, und als Folge davon führen sehr kleine Verdrehungswinkel ϑ zu einem großen Verschwenkwinkel β des freien Endes des Schwenkhebels 4 und damit auch des mit dem Permanentmagneten 8 versehenen Trägerelements 5.

Fig. 2B zeigt die Vorrichtung bei einem mittleren Drehmoment. Dabei ist der Schwenkhebel 4 aufgrund einer Relativverdrehung zwischen der Antriebsachse 1 und der Radnabe im Gegenuhrzeigersinn verschwenkt worden, und diese geringe Relativverdrehung hat mit der entsprechenden Übersetzung ein Verschwenken des Trägerelements 5 und damit der in diesem Trägerelement 5 befestigten Permanentmagnete im Uhrzeigersinn zur Folge.

Fig. 2C zeigt den Fall des Anliegens eines maximalen Dreh-bzw. Antriebsmoments. Dabei macht insbesondere ein Vergleich der Fig. 2A und 2C deutlich, daß die erfindungsgemäß vorgesehene mechanische Übersetzung der vom jeweiligen Antriebsmoment abhängigen Relativverdrehung zwischen Antriebsachse 1 und Radnabe 3 zu einer Multiplikation dieser Relativverdrehung führt, die ohne weiteres einem Faktor 5 bis 10 entsprechen kann.

Durch geeignete Ausgestaltung der mit dem Schwenkhebel 4 zusammenwirkenden Steuerprofilfläche 17 am Trägerelement 5 kann die jeweils gewünschte Übersetzungscharakteristik definiert vorgegeben und insbesondere auch linear gewählt werden.

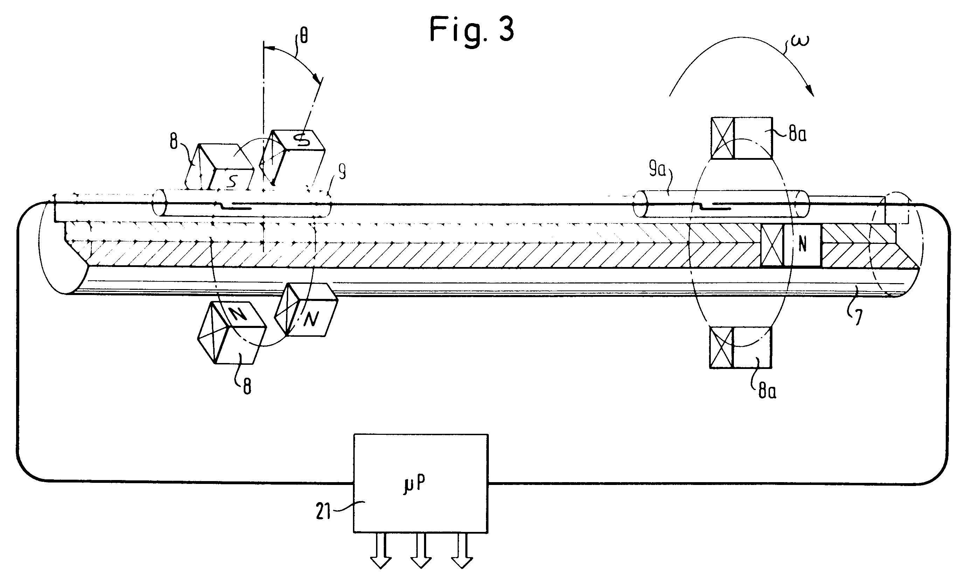

Die schematische Darstellung gemäß Fig. 3 zeigt die Auswertung der bei einer Anordnung der beschriebenen Art gewonnenen Signale. Die mit den Permanentmagneten 8, 8a zusammenwirkenden Reedkontakte 9, 9a sind elektrisch in Reihe geschaltet. Wird auf ein mit der erfindungsgemäßen Vorrichtung versehenes Rad kein Antriebsmoment ausgeübt, dann sind die von den Reedkontakten 9, 9a erzeugten Impulsfolgen in Phase. Wird auf die Antriebsachse ein Antriebsmoment aufgebracht, so ergibt sich zwischen den jeweils mit gleicher Winkelgeschwindigkeit ω umlaufenden Magneten 8, 8a eine Winkelversetzung und damit auch eine Versetzung der Einschalt- bzw. Ausschaltflanken der vom Reedkontakt 9 erzeugten Impulse bezüglich der entsprechenden Flanken der vom Reedkontakt 9a erzeugten und durch die fest montierten Magnete 8a bewirkten Impulse.
Die Auswertung dieser Impulsfolgen erfolgt vorzugsweise mittels eines Mikroprozessors 21.

## Patentansprüche

1. Vorrichtung zur Messung des auf ein antreibbares Rad, insbesondere auf das Antriebsrad eines Fahrrades ausgeübten Antriebsmomentes und/oder damit verbundener Größen,
bestehend aus einer bezüglich einer festen Achse (7) drehbar gelagerten Radnabe (3), einer koaxial zwischen fester Achse (7) und Radnabe (3) angeordneten Antriebsachse (1), einem am antriebsseitigen Ende zwischen Antriebsachse (1) und Radnabe (3) vorgesehenen, eine Relativverdrehung ermöglichenden Organ (18′) und einer am anderen Ende ausgebildeten Festverbindung (11) zwischen Antriebsachse (1) und Radnabe (3), einer Einrichtung zur Erfassung der vom Antriebsmoment abhängigen Relativverdrehung zwischen Radnabe (3) und Antriebsachse (1) sowie zur Erzeugung von zur Auswertung geeigneten, die Relativverdrehung repräsentierenden Signalen,
dadurch **gekennzeichnet**,
daß in einem Führungsraum (12) zwischen der festen Achse (7) und der Antriebsachse (1) ein um die feste Achse (7) verschwenkbares Trägerelement (5) für zumindest einen Meßwertgeber (8) vorgesehen ist,
daß dem Meßwertgeber (8) wenigstens ein die Verschwenkung des Trägerelements (5) erfassender Sensor (9) zugeordnet ist, und daß der Schwenkwinkel des Trägerelements (5) durch ein mechanisches, zwischen Radnabe (3) und Antriebsachse (1) wirksames und durch deren Relativverdrehung betätigtes Übersetzungsorgan (4) gesteuert ist.

2. Vorrichtung nach Anspruch 1,
dadurch **gekennzeichnet**,
daß der Sensor (9) bezüglich der festen Achse (7), der Antriebsachse (1) oder der Nabe (3) fixiert ist.

3. Vorrichtung nach Anspruch 1 oder 2,
dadurch **gekennzeichnet**,
daß zumindest ein weiteres Paar von Meßwertgeber (8a) und zugeordnetem Sensor (9a) außerhalb des Bereichs der Antriebsachse (1) an der Radnabe (3) bzw. an der festen Achse (7) vorgesehen ist.

4. Vorrichtung nach einem der vorhergehenden Ansprüche,
dadurch **gekennzeichnet**,
daß der Führungsraum (12) für das verschwenkbare Trägerelement (5) im Bereich des antriebsseitigen Endes der Antriebsachse (1) und insbesondere radial innerhalb des zwischen Radnabe (3) und Antriebsachse (1) vorgesehenen, eine Relativverdrehung ermöglichenden Organs (18′) gelegen ist.

5. Vorrichtung nach einem der vorhergehenden Ansprüche,
dadurch **gekennzeichnet**,
daß das eine Relativverdrehung ermöglichende Organ (18′) aus einer Anordnung von zwei durch verformbare Radialstege miteinander verbundenen konzentrischen Kunststoffringen oder einem Kugellager besteht.

6. Vorrichtung nach einem der vorhergehenden Ansprüche,
dadurch **gekennzeichnet**,
daß das verschwenkbare Trägerelement (5) ringscheibenförmig ausgebildet und durch eine Feder (6) in einer Richtung gegen das Übersetzungsorgan (4) vorgespannt ist.

7. Vorrichtung nach Anspruch 6,
dadurch **gekennzeichnet**,
daß das ringscheibenförmige Trägerelement (5) eine Ausnehmung (13) aufweist, in die das als Schwenkhebel ausgebildete Übersetzungsorgan (4) eingreift.

8. Vorrichtung nach Anspruch 7,
dadurch **gekennzeichnet**,
daß der Schwenkhebel (4) mit einem Ende an einer nabenfesten Achse (14) gelagert ist, eine Öffnung (15) in der Antriebsachse (1) durchsetzt und mit dem anderen Ende an dem Trägerelement (5) anliegt, und daß eine Begrenzungswand (16) der Öffnung (14) aufgrund der Federvorspannung des Trägerelements (5) stets an dem Schwenkhebel (4) anliegt und das Schwenkhebelbetätigungsorgan bildet.

9. Vorrichtung nach Anspruch 8,
dadurch **gekennzeichnet**,
daß das Lager (14) für den Schwenkhebel (4) in einem mit der Radnabe (3) fest verbundenen Führungsstück (2) ausgebildet und unmittelbar benachbart der Antriebsachse (1) angeordnet ist.

10. Vorrichtung nach einem der vorhergehenden Ansprüche,
dadurch **gekennzeichnet**,
daß diejenige Seitenfläche der Ausnehmung (13) im Trägerelement (5), an der der Schwenkhebel (4) anliegt, als Steuerprofilfläche (17) ausgebildet ist.

11. Vorrichtung nach Anspruch 10,
dadurch **gekennzeichnet**,
daß die Steuerprofilfläche (17) so gestaltet ist, daß eine lineare Übersetzungscharakteristik gegeben ist.

12. Vorrichtung nach einem der vorhergehenden Ansprüche,
dadurch **gekennzeichnet**,
daß das freie Ende des Schwenkhebels (4) eine einer Steuerflächenkontur entsprechende Formgebung besitzt.

13. Vorrichtung nach einem der vorhergehenden Ansprüche,
dadurch **gekennzeichnet**,
daß das Trägerelement (5) aus Kunststoff besteht.

14. Vorrichtung nach einem der vorhergehenden Ansprüche,
dadurch **gekennzeichnet**,
daß die Meßwertgeber (8, 8a) aus Permanentmagneten und die Sensoren (9, 9a) aus Reedkontakten bestehen.

15. Vorrichtung nach einem der vorhergehenden Ansprüche,
dadurch **gekennzeichnet**,
daß die zeitliche Versetzung der dem Öffnen oder Schließen der Reedkontakte (9, 9a) zugeordneten Signalflanken als das jeweilige Antriebsmoment repräsentierende Meßgröße verwendet ist.

16. Vorrichtung nach einem der vorhergehenden Ansprüche,
dadurch **gekennzeichnet**,
daß Meßwertgeber und Sensor aus einer insbesondere scheibenförmig ausgebildeten Widerstandsanordnung mit zugeordnetem Abgreifelement bestehen.

## Claims

1. Apparatus for the measurement of the drive torque exerted on a drivable wheel, in particular on the drive wheel of a bicycle, and/or of parameters associated with the drive torque, the apparatus comprising a wheel hub (3) which is rotatably journalled relative to a fixed axle (7); a drive axle (1) which is coaxially disposed between the fixed axle (7) and the wheel hub (3); a member (18') which permits a relative rotation disposed between the drive axle (1) and the wheel hub (3) at the drive side end; a fixed connection (11) formed at the other end between the drive axle (1) and the wheel hub (3); a device for detecting the relative rotation between the wheel hub (3) and the drive axle (1) which depends on the drive torque and also for generating signals representative of the relative rotation and suitable for evaluation,
characterised in that
a carrier element (5) for at least one measurement transducer (8) is provided in a guide space (12) between the fixed axle (7) and the drive axle (1) and is pivotable about the fixed axle (7); in that at least one sensor (9) which detects the pivoting of the carrier element (5) is associated with the measurement transducer (8); and in that the pivot angle of the carrier element (5) is controlled by a mechanical multiplier member (4) which acts between the wheel hub (3) and the drive axle (1) and is actuated by their relative rotation.

2. Apparatus in accordance with claim 1,
characterised in that
the sensor (9) is fixed relative to the fixed axle (7), the drive axle (1) or the hub (3).

3. Apparatus in accordance with claim 1 or claim 2,
characterised in that
at least one further pair comprising a measurement transducer (8a) and associated sensor (9a) is provided outside of the region of the drive axle (1) at the wheel hub (3) or at the fixed axle (7).

4. Apparatus in accordance with one of the preceding claims, characterised in that
the guide space (12) for the pivotable carrier element (5) is provided in the region of the drive side end of the drive axle (1), and in particular radially inside the member (18') which is provided between the wheel hub (3) and the drive axle (1) and which permits a relative rotation.

5. Apparatus in accordance with one of the preceding claims, characterised in that
the member (18') which permits a relative rotation comprises either an arrangement of two concentric plastic rings which are connected together by deformable radial webs or a ball-bearing.

6. Apparatus in accordance with one of the preceding claims, characterised in that
the pivotable carrier element (5) is of ring disc-like shape and is biased by a spring (6) in a direction towards the multiplier member (4).

7. Apparatus in accordance with claim 6,
characterised in that
the ring disc-like carrier (5) has a recess (13) into which the multiplier member (4) engages which is formed as a pivot lever.

8. Apparatus in accordance with claim 7,
characterised in that
the pivot lever (4) is journalled at one end at an axis (14) fixed relative to the hub, passes through an opening (15) in the drive axle (1) and contacts the carrier element (5) with its other end; and in that a boundary wall (16) of the opening (14) always contacts the pivot lever (4) as result of the spring bias of the carrier element (5) and forms the pivot lever actuating member.

9. Apparatus in accordance with claim 8,
characterised in that
the bearing (14) for the pivot lever (4) is formed in a guide piece (2) which is fixedly connected to the wheel hub (3) and is arranged directly adjacent the drive axle (1).

10. Apparatus in accordance with one of the preceding claims, characterised in that
the side surface of the recess (13) in the carrier element (5) which is contacted by the pivot lever (4) is formed as a profiled control surface (17).

11. Apparatus in accordance with claim 10,
characterised in that
the profiled control surface (17) is so laid out that a linear multiplication characteristic is present.

12. Apparatus in accordance with one of the preceding claims, characterised in that
the free end of the pivot lever (4) has a shape which corresponds to a contour of the control surface.

13. Apparatus in accordance with one of the preceding claims, characterised in that
the carrier element (5) consists of plastic.

14. Apparatus in accordance with one of the preceding claims, characterised in that
the measurement transducers (8, 8a) comprise permanent magnets and the sensors (9, 9a) comprise reed contacts.

15. Apparatus in accordance with one of the preceding claims, characterised in that
the timewise displacement of the signal flanks associated with the opening and closing of the reed contacts (9, 9a) is used as the measurement parameter representative for the particular drive moment.

16. Apparatus in accordance with one of the preceding claims, characterised in that
measurement transducers and sensors comprise a resistor arrangement in particular of disc-like shape with an associated tapping element.

## Revendications

1. Dispositif pour mesurer le couple d'entraînement appliqué à une roue entraînée, notamment à la roue motrice d'une bicyclette, et/ou de grandeurs connexes, composé d'un moyeu de roue (3) monté tournant par rapport à un axe (7) fixe, un arbre d'entraînement (1) qui est monté de façon coaxiale entre l'axe (7) fixe et le moyeu de roue (3), un organe (18') qui permet une rotation relative et est disposé entre l'arbre d'entraînement (1) et le moyeu de roue (3), à l'extrémité côté entraînement, une liaison (11) rigide entre l'arbre d'entraînement (1) et le moyeu (3) disposée à l'autre extrémité, un dispositif pour mesurer la rotation relative fonction du couple d'entraînement du moyeu de roue (3) par rapport à l'arbre d'entraînement (1) et pour produire des signaux représentatifs de la rotation relative adaptés pour le traitement, caractérisé par le fait qu'il est prévu dans une chambre de guidage (12), entre l'axe (7) fixe et l'arbre d'entraînement (1), un élément support (5) pivotant pour au moins un transmetteur de signaux (8), par le fait qu'au moins un détecteur (9) qui détecte le pivotement de l'élément support (5) est associé au transmetteur de signaux (8) et par le fait que l'angle de pivotement de l'élément support (5) est commandé par un organe de transmission (4) mécanique qui agit entre le moyeu de roue (3) et l'arbre d'entraînement (1) et est actionné par la rotation relative de ces derniers.

2. Dispositif selon la revendication 1, caractérisé par le fait que le détecteur (9) est immobile par rapport à l'axe (7) fixe, à l'arbre d'entraînement (1) ou au moyeu de roue (3).

3. Dispositif selon la revendication 1 ou 2, caractérisé par le fait qu'il est prévu au moins une paire supplémentaire de transmetteurs de signaux (8a) et de détecteurs (9a) en dehors de la zone de l'arbre d'entraînement (1) sur le moyeu de roue (3) ou sur l'axe (7) fixe.

4. Dispositif selon l'une des revendications précédentes, caractérisé par le fait que la chambre de guidage (12) pour l'élément support (5) est située dans la région de l'extrémité côté entraînement de l'arbre d'entraînement (1), notamment radialement à l'intérieur de l'organe (18') qui est prévu entre le moyeu de roue (3) et l'arbre d'entraînement (1) et permet une rotation relative.

5. Dispositif selon l'une des revendications précédentes, caractérisé par le fait que l'organe (18') permettant une rotation relative est formé par un agencement de deux bagues concentriques en matière plastique qui sont reliées entre elles par deux nervures radiales déformables ou par un roulement à billes.

6. Dispositif selon l'une des revendications précédentes, caractérisé par le fait que l'élément support (5) pivotant est agencé sous forme de disque annulaire et est appliqué avec précontrainte contre l'organe de transmission (4) par un ressort (6) qui agit dans une direction.

7. Dispositif selon la revendication 6, caractérisé par le fait que l'élément support (5) en forme de disque annulaire comporte un évidement (13) dans lequel pénètre l'organe de transmission (4) agencé sous forme de levier oscillant.

8. Dispositif selon la revendication 7, caractérisé par le fait que le levier oscillant (4) est monté à l'une de ses extrémités sur un axe (14) solidaire du moyeu, traverse une ouverture (15) dans l'arbre d'entraînement (1) et est appliqué par son autre extrémité sur l'élément support (5) et par le fait que par suite de la précontrainte élastique appliquée sur l'élément support (5) , une paroi (16) de limitation de l'ouverture (14) est appliquée en permanence contre le levier oscillant (4) et constitue l'organe d'actionnement dudit levier oscillant.

9. Dispositif selon la revendication 9, caractérisé par le fait que le palier (14) pour le levier oscillant (4) est agencé dans une partie de guidage (2) solidaire du moyeu de roue (3) et est disposé à proximité immédiate de l'arbre d'entraînement (1).

10. Dispositif selon l'une des revendications précédentes, caractérisé par le fait que la surface latérale de l'évidement (13) dans l'élément support (5) contre laquelle le levier oscillant (4) vient en appui est agencée sous forme de surface de commande (17) profilée.

11. Dispositif selon la revendication 10, caractérisé par le fait que la surface de commande (17) profilée est agencée de manière à obtenir une caractéristique de transmission linéaire.

12. Dispositif selon l'une des revendications précédentes, caractérisé par le fait que l'extrémité libre du levier oscillant (4) a une forme qui correspond au profil de la surface de commande.

13. Dispositif selon l'une des revendications précédentes, caractérisé par le fait que l'élément support (5) est en matière plastique.

14. Dispositif selon l'une des revendications précédentes, caractérisé par le fait que les transmetteurs de signaux (8, 8a) sont constitués par des aimants permanents et les détecteurs (9, 9a) par des contacts de Reed.

15. Dispositif selon l'une des revendications précédentes, caractérisé par le fait que l'on utilise le décalage dans le temps des flancs de signaux associés à l'ouverture ou à la fermeture des contacts de Reed (9, 9a) comme grandeur de mesure représentant le couple d'entraînement.

16. Dispositif selon l'une des revendications précédentes, caractérisé par le fait que le transmetteur de signaux et le détecteur sont constitués par un ensemble de résistances notamment en forme de disque avec un doigt de lecture associé.
